# EUROPEAN PATENT SPECIFICATION

(11) **EP 2 726 127 B1**
(45) Date of publication and mention of the grant of the patent: **15.02.2017**
(21) Application number: 12737349.6
(22) Date of filing: 18.06.2012
(51) Int. Cl.: A61M 16/00

(54) **APPARATUS FOR ASSISTING AIRWAY CLEARANCE**
VORRICHTUNG ZUR UNTERSTÜTZUNG EINER ATEMWEGSREINIGUNG
APPAREIL POUR ASSISTER LE DÉGAGEMENT DES VOIES RESPIRATOIRES

(30) Priority: 29.06.2011 US 201161502357 P
(43) Date of publication of application: 07.05.2014
(73) Proprietor: Koninklijke Philips N.V., 5656 AE Eindhoven (NL)
(72) Inventor: MCDANIEL, Christopher Wayne, 5656 AE Eindhoven (NL)
(74) Representative: de Haan, Poul Erik
(86) International application number: PCT/IB2012/053048
(87) International publication number: WO 2013/001398

(56) References cited:
- WO-A1-2011/010279
- WO-A2-2010/058308
- US-A1- 2007 199 566

## Description

### BACKGROUND OF THE INVENTION

### 1. Field of the Invention

The present invention pertains to an apparatus for assisting a subject in clearing his or her airway (i.e., coughing), and, in particular, to an in-exsufflation method and apparatus that includes an expiratory hold feature that is synchronized with a manually assisted cough.

### 2. Description of the Related Art

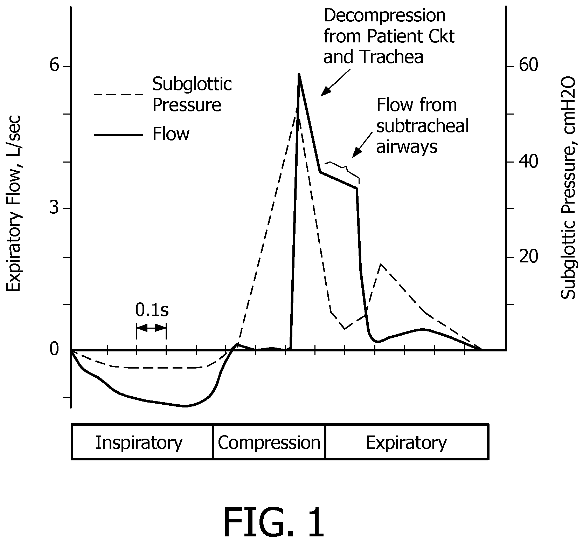

Coughing, also known as "airway clearance," is a normal function of everyday life for most people. A typical cough sequence for a healthy individual able to cough normally is graphed in FIG. 1. As seen in FIG. 1, during an inspiratory phase, inhaled air is drawn into the lungs slowly (e.g., at a rate of less than 1 LPS) through the trachea. Then, during a compression phase, the individual's glottis, which covers the trachea, closes and the individual's expiratory muscles contract. As seen in FIG. 1, the closing of the glottis coupled with the contraction of the expiratory muscles increases the subglottic pressure (i.e., the pressure in the trachea below the closed glottis) of the individual. Finally, when the subglottic pressure reaches a certain level, the individual enters an expiratory phase of the cough. During the expiratory phase, the glottis opens and the initial flow outward is due to the decompression of the air in the trachea. Thereafter, the lungs continue to empty at a rate of roughly 4 LPS until the lungs are sufficiently decompressed.

Some people, due to injury, disease, or even thoracic surgery, have severely compromised or disabled glottic function, and, as a result, find it difficult or impossible to cough effectively on their own. This is largely due to the fact that such people are unable to increase their subglottic pressure during the compression phase of cough (FIG. 1) in preparation for the rapid decompression during the expiratory phase. For these people, assisted or artificial airway clearance is prescribed.

Artificial airway clearance can be achieved via many known methods. One such method employs the use of a device known as a mechanical in-exsufflator (MI-E). An MI-E is a medical device that delivers positive airway pressure through the mouth, nose, or a tracheostomy to gently fill the lungs to capacity (a process known as insufflation). It then very abruptly reverses pressure, which generates an explosive expiratory flow, mimicking a cough (a process known as exsufflation). Thus, MI-E devices attempt to generate effective expiratory flow rates through a combination of hyperinflation during the inspiratory phase and negative pressure during the expiratory phase.

In addition, in some cases, a physician or caregiver may wish to increase the effectiveness of the MI-E therapy by augmenting it with a manual-assist cough technique, such as a manually applied abdominal thrust. In such a case, the caregiver would skillfully position his or her hand(s) on the patient's abdomen and apply force in synch with the initiation of the exsufflation phase of the therapy (i.e., the force is applied in synch with the initiation and application of the reverse exsufflation pressure). This applied abdominal thrust will further increase the peak cough flow (PCF), and thus improve the effective mobilization of the patient's secretions.

While prior art artificial airway clearance methods as just described have been proven to be effective for many individual, there is room for improvement in this area. In particular, there is a need for an artificial airway clearance method that facilitates even higher PCF levels during treatment without requiring the MI-E device to deliver higher negative exsufflation pressures to the patient.

US 2007/0199566 A1 discloses a respiratory device for performing mechanical ventilation and/or inexsufflation. The respiratory device includes a mechanical medical ventilator, a sensor, a display and a processor. The mechanical medical ventilator assists a patient with the respiratory cycle. The sensor measures an intra-thoracic respiratory parameter during the respiratory cycle. The display displays a graphical representation that dynamically depicts at least one of a patient's lung or thorax based on the intra-thoracic respiratory parameter in real-time during the respiratory cycle. The processor updates the graphical representation on the display in real-time based on the respiratory parameter. The processor updates the graphical representation to depict at least one of an expansion or contraction of at least one of the lung or thorax during the respiratory cycle.

### SUMMARY OF THE INVENTION

It is an object of the present invention to provide an apparatus for assisting a patient with airway clearance that does not suffer from the disadvantages associated with conventional devices techniques.

This object is solved according to claim 1 by an apparatus for assisting a patient with airway clearance is provided that includes a gas flow generating component structured to selectively generate a positive pressure insufflation gas flow and a negative pressure exsufflation gas flow, a patient circuit having a patient interface device operatively coupled to the gas flow generating component, and a controller operatively coupled to the gas flow generating component. The controller is structured to: (i) cause the gas flow generating component to provide the positive pressure insufflation gas flow to the patient through the patient circuit during an insufflation phase,
(ii) determine that the insufflation phase is complete, (iii) responsive to determining that the insufflation phase is complete, cause the patient circuit to enter an operating condition wherein the patient, when coupled to the patient interface device, will be in an expiratory hold condition wherein the patient is prevented from exhaling, (iv) responsive to determining that a certain condition has been met, cause the patient circuit to no longer be in the operating condition such that the expiratory hold condition is terminated, and (v) following termination of the expiratory hold condition, cause the gas flow generating component to provide the negative pressure exsufflation gas flow to the patient through the patient circuit during an exsufflation phase.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a graph illustrating a typical cough sequence in a healthy individual;
FIG. 2 is a schematic diagram of an MI-E device that may be used to implement an in-exsufflation method according to one exemplary embodiment;
FIG. 3 is a schematic diagram of an MI-E device that may be used to implement an in-exsufflation method according to an alternative exemplary embodiment;
FIG. 4 is a flowchart illustrating an in-exsufflation method according to an exemplary embodiment;
FIG. 5 is a flowchart illustrating an in-exsufflation method according to one particular exemplary embodiment;
FIG. 6 is a flowchart illustrating an in-exsufflation method according to an alternative particular exemplary embodiment; and
FIGS. 7 and 8 are "idealized" graphs of time-based pressure and flow waveforms, respectively, that illustrate the benefits of the present invention.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

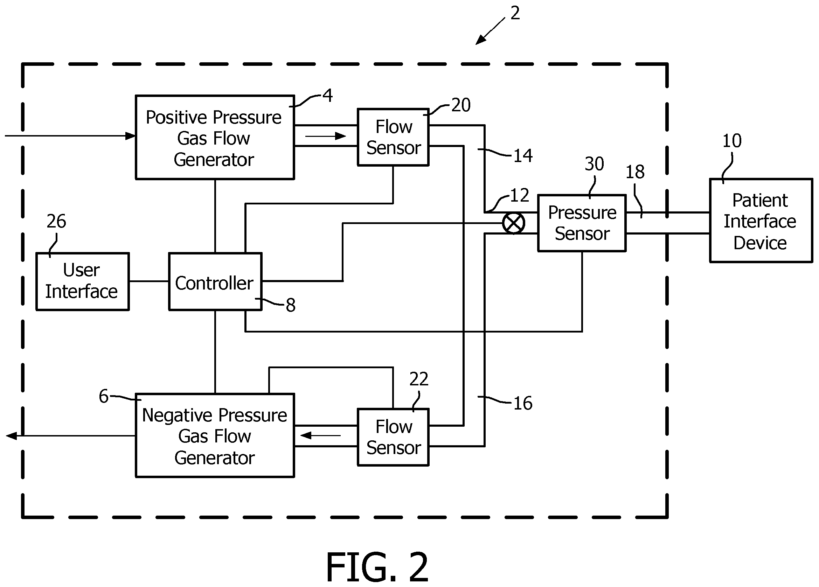

FIG. 2 is a schematic diagram of an MI-E device 2 that may be used to implement the in-exsufflation method according to one exemplary embodiment. As seen in FIG. 2, MI-E device 2 includes a positive pressure gas flow generator 4 and a negative gas flow generator 6, each of which is operatively coupled to and controlled by a controller 8. Positive pressure gas flow generator 4 is structured to generate airflow under positive-pressure for use in insufflation of a patient as described herein. Positive pressure gas flow generator 4 may comprise a device such as a centrifugal blower (compressor), turbine, piston, bellows or another suitable apparatus known in the art for generating airflow under positive pressure. For example, positive pressure gas flow generator 4 may comprise, for example, a blower used in a conventional CPAP or bi-level pressure support device.

Negative pressure gas flow generator 6 is structured to generate airflow under negative-pressure for use in exsufflation of a patient as described herein. Negative pressure gas flow generator 6 may, like positive pressure gas flow generator 4, may comprise a device such as a centrifugal blower (compressor), turbine, piston, bellow or another suitable apparatus known in the art for generating airflow under negative pressure. In the exemplary embodiment, positive pressure gas flow generator 4 and negative pressure gas flow generator 6 each includes a valve (not shown) controlled by controller 8 that functions as a pressure controller or flow controller for positive pressure gas flow generator 4 and negative pressure gas flow generator 6 as the case may be.

It should be apparent that other techniques for controlling the pressure or the flow delivered by positive pressure gas flow generator 4 and negative pressure gas flow generator 6, such as varying the blower speed, either alone or in combination with a pressure control valve, are contemplated by the present disclosure. In addition, in use as described herein, when positive pressure gas flow generator 4 is providing a positive pressure flow, the valve associated with negative pressure gas flow generator 6 will be caused to be closed, and similarly, when negative pressure gas flow generator 6 is providing a negative pressure flow, the valve associated with positive pressure gas flow generator 4 will be caused to be closed.

Controller 8 includes a processing portion which may be, for example, a microprocessor, a microcontroller or some other suitable processing device, and a memory portion that may internal to the processing portion or operatively coupled to the processing portion and that provides a storage medium for data and software executable by the processing portion for controlling the operation of MI-e device 2 as described in greater detail herein.

MI-E device 2 also includes a patient interface device 10 that is coupled to both positive pressure gas flow generator 4 and negative pressure gas flow generator 6 by a Y-shaped delivery conduit 12 having a positive pressure branch 14 (connected to positive pressure gas flow generator 4), a negative pressure branch 16 (connected to negative pressure gas flow generator 6), and a common portion 18 (connected to patient interface device 10). Patient interface device 10, which may be a facemask, an endotracheal tube, a tracheostomy tube, or any other suitable means known in the art for establishing an interface between a patient and another medical device, interfaces positive pressure gas flow generator 4 and negative pressure gas flow generator 6 with a patient.

In the illustrated embodiment, a flow sensor 20 is provided in positive pressure branch 14 for measuring the gas flow rate therein and a flow sensor 22 is provided in negative pressure branch 16 for measuring the gas flow rate therein. The function of flow sensors 20, 22 in one particular, non-limiting exemplary embodiment is described elsewhere herein. As will be appreciated, in the event that that particular, non-limiting exemplary embodiment is not implemented, flow sensors 20, 22 may be omitted. Furthermore, in the illustrated embodiment, a valve 24, controlled by controller 8, is provided in common portion 18. Rather than being automatically controlled by controller 8, valve 24 may be manually controlled by a user of MI-E device 2, such as a clinician or caregiver. The function of valve 24 in one particular, non-limiting exemplary embodiment is described elsewhere herein. As will be appreciated, in the event that that particular, non-limiting exemplary embodiment is not implemented, valve 24 may be omitted.

MI-E device 2 also includes a user interface 26 for setting various parameters used by MI-E device 2, as well as for displaying and outputting information and data to a user, such as a clinician or caregiver.

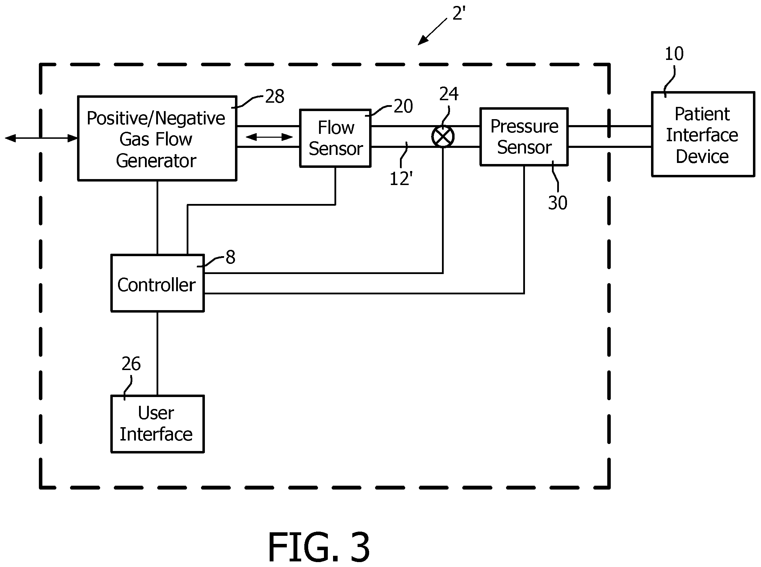

FIG. 3 is a schematic diagram of an MI-E device 2' that may be used to implement the in-exsufflation method according to an alternative exemplary embodiment. MI-E device 2' includes many of the same components as MI-E device 2, and like parts are labeled with like reference numerals. However, as seen in FIG. 3, rather than including a separate positive pressure gas flow generator 4 and negative pressure gas flow generator 6, MI-E device 2' includes a single component, positive/negative pressure gas flow generator 28, that is, under control of controller 8, structured to generate both airflow under positive-pressure for use in insufflation of a patient as described herein and airflow under negative-pressure for use in exsufflation of a patient as described herein. Positive/negative pressure gas flow generator 28 may comprise a device such as a centrifugal blower (compressor), turbine, piston, bellow or another suitable apparatus known in the art for selectively generating airflow under both positive and negative pressures. In the exemplary embodiment, positive/negative pressure gas flow generator 28 is a centrifugal blower that includes an arrangement of valves that is used to regulate the pressure that is delivered it to the patient. During the insufflation phase, the valves direct the outlet of the blower to the patient in order to deliver positive pressure. During the exsufflation phase, the valves direct the inlet side of the blower to the patient in order to deliver negative pressure.

According to an aspect of the method, the patient is placed in an expiratory hold condition. In that condition, the patient is prevented from exhaling through patient interface device 10. The patient's glottis may or may not close during the expiratory hold condition depending on the condition of the patient. In one embodiment, the expiratory hold condition is achieved by closing valve 24 such that the circuit including patient interface device 10 is substantially physically occluded. In another embodiment, the expiratory hold condition is achieved by increasing the positive supply pressure provided to the patient such that exhalation is inhibited. It will be understood that alternative manners and mechanisms for placing a patient in an expiratory hold condition are possible and contemplated within the scope of the present disclosure.

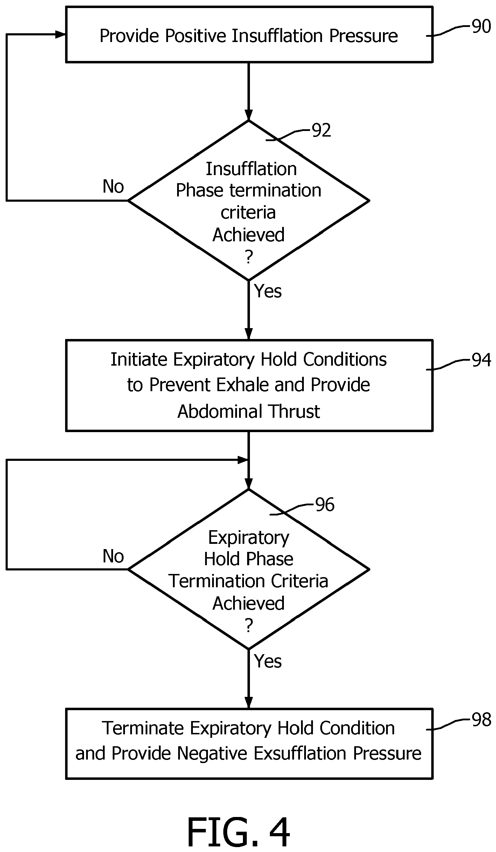

FIG. 4 is a flowchart illustrating an in-exsufflation method according to an exemplary embodiment. The method shown in FIG. 4 may be performed using a suitable MI-E device, wherein certain portions of the method are implemented as one or more routines executable by the controller of the MI-E device for controlling the MI-E device as described. For purposes of illustrating, the method of FIG. 4 will be described as being implemented in either MI-E device 2 or MI-E device 2'.

Referring to FIG. 4, the method begins at step 90, wherein controller 8 causes a positive insufflation pressure to be provided to the patient (to inflate the patient's lungs) through patient interface device 10 by controlling either positive pressure gas flow generator 4 (in the case of MI-E device 2) or positive/negative pressure gas flow generator 28 (in the case of MI-E device 2'). Next, at step 92, a determination is made by controller 8 as to whether a predetermined insufflation phase termination criteria (e.g. a predetermined inflation volume, inflation pressure, inflation time, or inflation flow rate) has been achieved. If the answer at step 92 is no, then the method returns to step 90 and insufflation continues.

If, however, the answer at step 92 is yes, then, at step 94, controller 8 causes an expiratory hold condition, as described elsewhere herein, to be initiated. Also at step 94, during the expiratory hold condition, the patient's clinician or caregiver commences a manual abdominal thrust on the patient, which results in a rapid increase in the patient's subglottic pressure. In the exemplary embodiment, the commencement of the manual abdominal thrust is time synchronized with the successful establishment of the expiratory hold condition (i.e., it is commenced as soon as the expiratory hold condition is achieved). In one particular embodiment, controller 8 is structured to cause user interface 28 to provide a user perceptible indicator (e.g., an audible or visual indicator) once the expiratory hold condition is achieved to let the clinician or caregiver know that her or she can commence the abdominal thrust.

Next, at step 96, a determination is made by controller 8 as to whether a predetermined expiratory hold phase termination criteria (e.g., a time threshold, a pressure threshold or activation of a manual switch) has been achieved. If the answer at step 96 is no, then the method returns to step 96 and continues to wait for the predetermined expiratory hold phase termination criteria to be achieved. If the answer at step 96 is yes, meaning the predetermined expiratory hold phase termination criteria has been achieved, then the method proceeds to step 98.

At step 98, controller 8 terminates the expiratory hold condition (e.g., the increased positive pressure is removed or valve 24 is opened) and then causes a negative insufflation pressure to be provided to the patient (to deflate the patient's lungs) through patient interface device 10 by controlling either negative pressure gas flow generator 4 (in the case of MI-E device 2) or positive/negative pressure gas flow generator 28 (in the case of MI-E device 2'). According to an aspect, because a higher subglottic pressure was generated as a result of the expiratory hold feature (combined with the abdominal thrust), a higher PCF will be achieved during the exsufflation phase (step 98). This higher PCF will in turn result in more effective secretion mobilization in the patient.

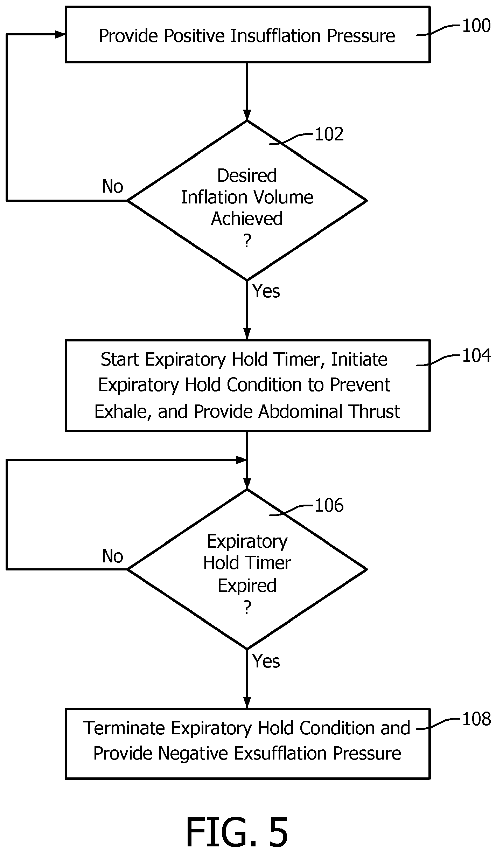

FIG. 5 is a flowchart illustrating an in-exsufflation method according to one particular exemplary embodiment. The method shown in FIG. 5, like the method of FIG. 4, may be performed using a suitable MI-E device, wherein certain portions of the method are implemented as one or more routines executable by the controller of the MI-E device for controlling the MI-E device as described. For purposes of illustrating, the method of FIG. 5 will be described as being implemented in either MI-E device 2 or MI-E device 2'.

Referring to FIG. 5, the method begins at step 100, wherein controller 8 causes a positive insufflation pressure to be provided to the patient (to inflate the patient's lungs) through patient interface device 10 by controlling either positive pressure gas flow generator 4 (in the case of MI-E device 2) or positive/negative pressure gas flow generator 28 (in the case of MI-E device 2'). Next, at step 102, a determination is made by controller 8 as to whether a predetermined desired inflation volume of the patient's lungs has been achieved. In the exemplary embodiment, the actual inflation volume of the patient's lungs is determined based on flow measurements made by flow sensor 20 in a well known manner. The particular desired inflation volume may be set by a user, such as a clinician or caregiver, using user interface 26. In one exemplary, non-limiting embodiment, the desired inflation volume is the maximum insufflation capacity (MIC) of the patient. If the answer at step 102 is no, then the method returns to step 100 and insufflation continues.

If, however, the answer at step 102 is yes, then, at step 104, controller 8 does two things: (i) it starts a timer, referred to as an expiratory hold timer, and (ii) it causes an expiratory hold condition, as described elsewhere herein, to be initiated. The expiratory hold timer is a timer that determines how long (some predetermined fixed time period) the patient will be kept in the expiration hold condition. The particular duration of the expiratory hold timer may be set by a user, such as a clinician or caregiver, using user interface 26. In one exemplary, non-limiting embodiment, the duration of the expiratory hold timer is one second.

Also at step 104, during the expiratory hold condition, the patient's clinician or caregiver commences a manual abdominal thrust on the patient, which results in a rapid increase in the patient's subglottic pressure. In the exemplary embodiment, the commencement of the manual abdominal thrust is time synchronized with the successful establishment of the expiratory hold condition (i.e., it is commenced as soon as the expiratory hold condition is achieved). In one particular embodiment, controller 8 is structured to cause user interface 28 to provide a user perceptible indicator (e.g., an audible or visual indicator) once the expiratory hold condition is achieved to let the clinician or caregiver know that her or she can commence the abdominal thrust.

Next, at step 106, a determination is made by controller 8 as to whether the expiratory hold timer has expired. If the answer is no, then the method returns to step 106 and continues to wait for the expiratory hold timer to expire. If the answer at step 106 is yes, then the method proceeds to step 108. At step 108, controller 8 terminates the expiratory hold condition (e.g., the increased positive pressure is removed or valve 24 is opened) and then causes a negative insufflation pressure to be provided to the patient (to deflate the patient's lungs) through patient interface device 10 by controlling either negative pressure gas flow generator 4 (in the case of MI-E device 2) or positive/negative pressure gas flow generator 28 (in the case of MI-E device 2'). As described elsewhere herein, because a higher subglottic pressure was generated as a result of the expiratory hold feature (combined with the abdominal thrust), a higher PCF will be achieved during the exsufflation phase (step 108). This higher PCF will in turn result in more effective secretion mobilization in the patient.

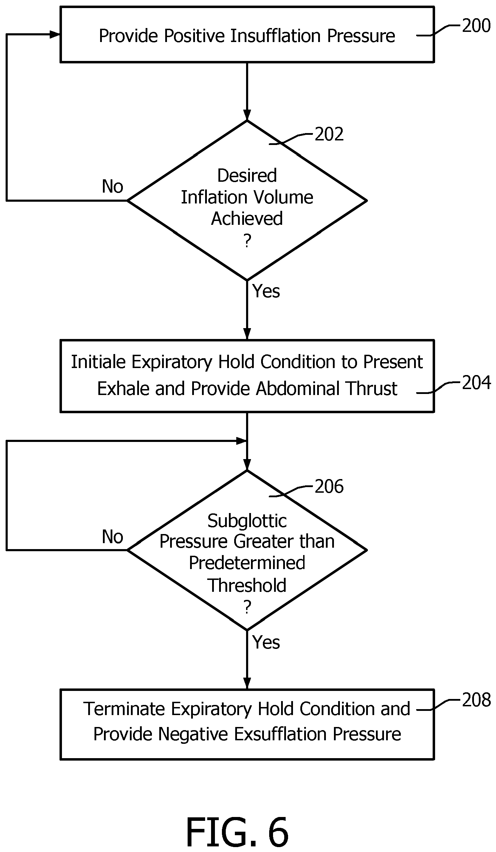

FIG. 6 is a flowchart illustrating an in-exsufflation method according to another particular exemplary embodiment. The method shown in FIG. 6, like the methods of FIGS. 4 and 5, may be performed using a suitable MI-E device, wherein certain portions of the method are implemented as one or more routines executable by the controller of the MI-E device for controlling the MI-E device as described. For purposes of illustrating, the method of FIG. 6 will be described as being implemented in either MI-E device 2 or MI-E device 2'.

Referring to FIG. 6, the method begins at step 200, wherein controller 8 causes a positive insufflation pressure to be provided to the patient (to inflate the patient's lungs) through patient interface device 10 by controlling either positive pressure gas flow generator 4 (in the case of MI-E device 2) or positive/negative pressure gas flow generator 28 (in the case of MI-E device 2'). Next, at step 202, a determination is made by controller 8 as to whether a predetermined desired inflation volume, as described elsewhere herein, has been achieved. If the answer at step 202 is no, then the method returns to step 200 and insufflation continues.

If, however, the answer at step 202 is yes, then, at step 204, controller 8 causes an expiratory hold condition, as described elsewhere herein, to be initiated. As described in detail below, in the present embodiment, the duration of the expiratory hold condition is not a fixed time period (as was the case in the method of FIG. 5), but instead is determined based on the patient's subglottic pressure. Also at step 204, during the expiratory hold condition, the patient's clinician or caregiver commences a manual abdominal thrust on the patient, which results in a rapid increase in the patient's subglottic pressure. In the exemplary embodiment, the commencement of the manual abdominal thrust is time synchronized with the successful establishment of the expiratory hold condition (i.e., it is commenced as soon as the expiratory hold condition is achieved). As described elsewhere herein, in one particular embodiment, controller 8 is structured to cause user interface 28 to provide a user perceptible indicator (e.g., an audible or visual indicator) once the expiratory hold condition is achieved to let the clinician or caregiver know that her or she can commence the abdominal thrust.

Next, at step 206, a determination is made by controller 8 as to whether the patient's subglottic pressure has exceeded a predetermined threshold pressure level. In the exemplary embodiment, for this purpose, the patient's subglottic pressure is measured by a pressure sensor 30 that measures gauge pressure at the exit of MI-E device 2 or MI-E device 2' (just before entering the patient tube connected to patient interface device 10). This sensor can be used to measure/calculate subglottic pressure with certain considerations in mind, depending on where you are in the time-based therapy profile. During the portions of the therapy when there is essentially zero flow, then the pressure measured by the sensor is arithmetically equivalent to the subglottic pressure in the patient's lungs. During the portions of the therapy when there is flow going through the system, the pressure measured by the sensor is a combination of subglottic pressure in the patient's lungs and the resistance of the patient circuit and patient airway structures leading up to the lungs. In this latter case, a mathematical model can be incorporated into the software algorithms, allowing the device to calculate subglottic pressure.

Referring again to step 206, the predetermined threshold pressure level may be set by a user, such as a clinician or caregiver, using user interface 26. In one exemplary, non-limiting embodiment, the predetermined threshold pressure level is 5 cmH2O above the pressure at the beginning of the expiratory hold phase. If the answer at step 206 is no, then the method returns to step 106 and continues to wait for the subglottic pressure to rise. If the answer at step 206 is yes, meaning the subglottic pressure has exceeded the predetermined threshold, then the method proceeds to step 208.

At step 208, controller 8 terminates the expiratory hold condition (e.g., the increased positive pressure is removed or valve 24 is opened) and then causes a negative insufflation pressure to be provided to the patient (to deflate the patient's lungs) through patient interface device 10 by controlling either negative pressure gas flow generator 4 (in the case of MI-E device 2) or positive/negative pressure gas flow generator 28 (in the case of MI-E device 2'). As described elsewhere herein, because a higher subglottic pressure was generated as a result of the expiratory hold feature (combined with the abdominal thrust), a higher PCF will be achieved during the exsufflation phase (step 208). This higher PCF will in turn result in more effective secretion mobilization in the patient.

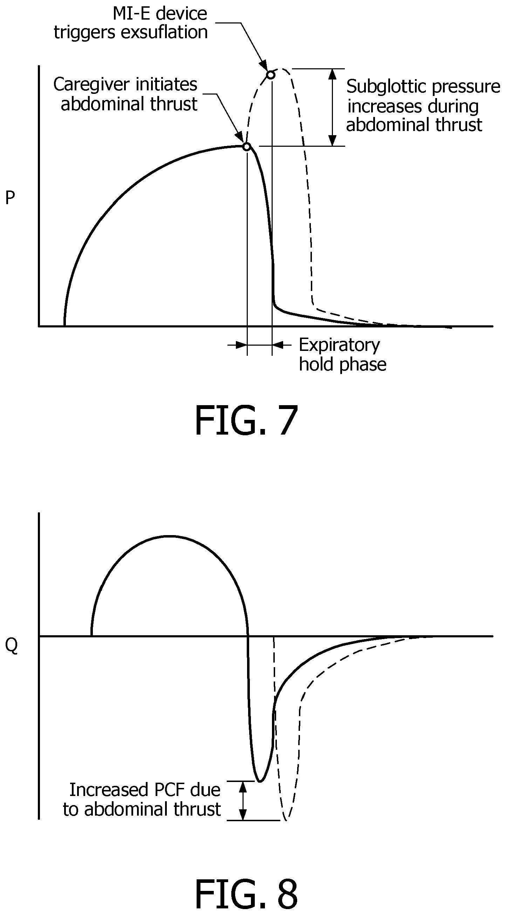

FIGS. 7 and 8 are "idealized" graphs of time-based pressure and flow waveforms, respectively, that illustrate the benefits of the present invention (solid lines are waveforms and generated based on use of prior art in-exsufflation methodology without an abdominal thrust and dotted lines are waveforms generated based on use of the methodology of the present invention). In FIG. 7, the pressure provided on the Y-axis is total pressure (subglottic + any flow resistance). Also in Fig. 7, in the prior art waveform (solid lines), the exsufflation phase begins at the point where the waveform starts heading back toward the x-axis. Note, the two waveforms in FIG. 7 are aligned with one another prior to the left of the first expiratory hold phase line.

In FIG. 8, the dotted line waveform is aligned with the prior art waveform up to the point that flow crosses the x-axis. Then, there is a period of time, during the abdominal thrust phase, when the dotted line waveform remains zero. Next, during the exsufflation phase, the dotted line waveform resumes a shape similar to that of the prior art. However, as result of the methodology of the invention, the dotted line waveform exhibits an increased peak (negative) flow. Thus, as seen in FIG. 7, the present invention provides increased subglottic pressure after insufflation as compared to the prior art, and as seen in FIG. 8, the present invention provides increased PCF after insufflation as compared to the prior art.

Furthermore, as described herein, flow sensors 20, 22 are positioned inside MI-E device 2 or MI-E device 2', between the pressure/flow generator and the location of pressure sensor 30. The output of pressure sensor 30 is used by the software as the primary signal to control the therapy delivered by the device. However, in practice, this signal can be somewhat unstable, often resulting in premature, late, or even false triggering of the various phases of therapy. In one particular embodiment, by adding a flow signal from flow sensor 20 and/or 22 to the control algorithms, the system can often minimize the likelihood of this occurring. For example, if the system monitors a sudden drop in pressure, but the slope of the flow signal is negative, then the system can intelligently determine that it is not a good time to trigger a new breath. In an alternative embodiment, flow sensor 20, or 22 may be positioned inside MI-E device 2 or MI-E device 2' between valve 24 and the location of pressure sensor 30.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. An apparatus (2, 2') for assisting a patient with airway clearance, comprising:
a gas flow generating component (4, 6, 28) structured to selectively generate a positive pressure insufflation gas flow and a negative pressure exsufflation gas flow;
a patient circuit having a patient interface (10) device operatively coupled to the gas flow generating component; and
a controller (8) operatively coupled to the gas flow generating component, the controller being structured to: (i) cause the gas flow generating component to provide the positive pressure insufflation gas flow to the patient through the patient circuit during an insufflation phase, (ii) determine that the insufflation phase is complete, and (v) following a termination of an expiratory hold condition, cause the gas flow generating component to provide the negative pressure exsufflation gas flow to the patient through the patient circuit during an exsufflation phase,
**characterized in that** the controller is further structured to: (iii) responsive to determining that the insufflation phase is complete, cause the patient circuit to enter an operating condition wherein the patient, when coupled to the patient interface device, will be in the expiratory hold condition wherein the patient is prevented from exhaling, (iv) responsive to determining that a certain condition has been met, cause the patient circuit to no longer be in the operating condition such that the expiratory hold condition is terminated.

2. The apparatus according to claim 1, further comprising a flow sensor (20) provided in the patient circuit, wherein the controller is structured to measure an inflation lung volume of the patient based on an output of the sensor and that the insufflation phase is complete when the inflation lung volume reaches a certain level.

3. The apparatus according to claim 1, wherein the certain condition is a predetermined fixed amount of time elapsing after the patient circuit has been caused to enter the operating condition.

4. The apparatus according to claim 3, further comprising a pressure sensor (30) structured to determine a subglottic pressure of the patient while the patient is in the expiratory hold condition, wherein certain condition is the subglottic pressure exceeding a predetermined threshold level.

5. The apparatus according to claim 1, wherein the causing the patient circuit to enter the operating condition includes causing the gas flow generating component to provide an increased positive pressure insufflation gas flow to the patient, the increased positive pressure insufflation gas flow having a pressure that is greater than a pressure of the positive pressure insufflation gas flow provided during the insufflation phase.

6. The apparatus according to claim 1, further comprising a valve (24) provided in the patient circuit, wherein the causing the patient circuit to enter the operating condition includes closing the valve to causing the patient circuit to be at least substantially physically occluded.

7. The apparatus according to claim 1, wherein the gas flow generating component comprises a positive pressure gas flow generator (4) structured to generate the positive pressure insufflation gas flow and a separate negative pressure gas flow generator (6) structured to generate the negative pressure exsufflation gas flow.

8. The apparatus according to claim 1, wherein the gas flow generating component comprises a gas flow generator (28) structured to generate both the positive pressure insufflation gas flow and the negative pressure exsufflation gas flow.

## Patentansprüche

1. Vorrichtung (2, 2') zur Unterstützung eines Patienten bei der Atemwegsreinigung, wobei die Vorrichtung Folgendes umfasst:
eine Gasströmungserzeugungskomponente (4, 6, 28), die konstruiert ist, um selektiv eine Insufflationsgasströmung mit positivem und eine Exsufflationsgasströmung mit negativem Druck zu erzeugen;
einen Patientenkreislauf mit einer Patientenschnittstellenvorrichtung (10), die betriebsfähig mit der Gasströmungserzeugungskomponente gekoppelt ist; und
eine Steuereinheit (8), die betriebsfähig mit der Gasströmungserzeugungskomponente gekoppelt ist, wobei die Steuereinheit konstruiert ist, um: (i) die Gasströmungserzeugungskomponente zu veranlassen, dem Patienten während einer Insufflationsphase über den Patientenkreislauf die Insufflationsgasströmung mit positivem Druck bereitzustellen, (ii) zu ermitteln, dass die Insufflationsphase abgeschlossen ist, und (v) nach der Beendigung eines exspiratorischen Haltezustands die Gasströmungserzeugungskomponente zu veranlassen, dem Patienten während einer Exsufflationsphase über den Patientenkreislauf die Exsufflationsgasströmung mit negativem Druck bereitzustellen,
**dadurch gekennzeichnet, dass** die Steuereinheit weiterhin konstruiert ist, um (iii) in Reaktion darauf, dass ermittelt wird, dass die Insufflationsphase abgeschlossen ist, den Patientenkreislauf zu veranlassen, in einen Betriebszustand einzutreten, in dem sich der Patient, wenn er mit der Patientenschnittstellenvorrichtung gekoppelt ist, in dem exspiratorischen Haltezustand befindet, in dem der Patient am Ausatmen gehindert wird, (iv) in Reaktion darauf, dass ermittelt wird, dass eine bestimmte Bedingung erfüllt ist, den Patientenkreislauf zu veranlassen, sich nicht mehr in dem Betriebszustand zu befinden, so dass der exspiratorische Haltezustand beendet wird.

2. Vorrichtung nach Anspruch 1, weiterhin umfassend einen in dem Patientenkreislauf vorgesehenen Durchflusssensor (20), wobei die Steuereinheit konstruiert ist, um ein Inflationslungenvolumen des Patienten basierend auf einer Ausgabe des Sensors zu messen, und dass die Insufflationsphase abgeschlossen ist, wenn das Inflationslungenvolumen ein bestimmtes Niveau erreicht hat.

3. Vorrichtung nach Anspruch 1, wobei die bestimmte Bedingung eine vorgegebene feste Zeitdauer ist, die abläuft, nachdem der Patientenkreislauf veranlasst wurde, in den Betriebszustand einzutreten.

4. Vorrichtung nach Anspruch 3, weiterhin umfassend einen Drucksensor (30), der konstruiert ist, um einen subglottischen Druck des Patienten zu ermitteln, während sich der Patienten in dem exspiratorischen Haltezustand befindet, wobei die bestimmte Bedingung darin besteht, dass der subglottische Druck einen vorgegebenen Schwellenwert überschreitet.

5. Vorrichtung nach Anspruch 1, wobei das Veranlassen des Patientenkreislaufs, in den Betriebszustand einzutreten, das Veranlassen der Gasströmungserzeugungskomponente umfasst, dem Patienten eine Insufflationsgasströmung mit erhöhtem positivem Druck bereitzustellen, wobei die Insufflationsgasströmung mit erhöhtem positivem Druck einen Druck hat, der größer ist als ein Druck der Insufflationsgasströmung mit positivem Druck, die während der Insufflationsphase bereitgestellt wird.

6. Vorrichtung nach Anspruch 1, weiterhin umfassend ein in dem Patientenkreislauf vorgesehenes Ventil (24), wobei das Veranlassen des Patientenkreislaufs, in den Betriebszustand einzutreten, das Schließen des Ventils umfasst, um den Patientenkreislauf zu veranlassen, zumindest im Wesentlichen physikalisch verschlossen zu sein.

7. Vorrichtung nach Anspruch 1, wobei die Gasströmungserzeugungskomponente eine Gasströmungsgenerator (4) mit positivem Druck umfasst, der konstruiert ist, um die Insufflationsgasströmung mit positivem Druck zu erzeugen, und einen separaten Gasströmungsgenerator (6) mit negativem Druck umfasst, der konstruiert ist, um die Exsufflationsgasströmung mit negativem Druck zu erzeugen.

8. Vorrichtung nach Anspruch 1, wobei die Gasströmungserzeugungskomponente einen Gasströmungsgenerator (28) umfasst, der konstruiert ist, um sowohl Insufflationsgasströmung mit positivem Druck als auch Exsufflationsgasströmung mit negativem Druck zu erzeugen.

## Revendications

1. Appareil (2, 2') pour assister un patient dans le dégagement de ses voies respiratoires, comprenant :
un composant générateur de flux de gaz (4, 6, 28) structuré pour générer sélectivement un flux de gaz d'insufflation sous pression positive et un flux de gaz d'exsufflation sous pression négative ;
un circuit de patient ayant un dispositif d'interface patient (10) couplé en service au composant générateur de flux de gaz ; et
un dispositif de commande (8) couplé en service au composant générateur de flux de gaz, le dispositif de commande étant structuré pour : (i) amener le composant générateur de flux de gaz à fournir le flux de gaz d'insufflation sous pression positive au patient via le circuit de patient au cours d'une phase d'insufflation, (ii) déterminer que la phase d'insufflation est terminée et (v), à l'achèvement d'un état de plateau expiratoire, amener le composant générateur de flux de gaz à fournir le flux de gaz d'exsufflation sous pression négative au patient via le circuit de patient au cours d'une phase d'exsufflation,
**caractérisé en ce que** le dispositif de commande est en outre structuré pour :
(iii), à la suite de la détermination que la phase d'insufflation est terminée, amener le circuit de patient à entrer dans un état opératoire dans lequel le patient, lorsqu'il est couplé au dispositif d'interface patient, sera dans l'état de plateau expiratoire dans lequel le patient est empêché d'exhaler, (iv), à la suite de la détermination qu'un certain état a été rencontré, amener le circuit de patient à ne plus être dans l'état opératoire de sorte que l'état de plateau expiratoire soit achevé.

2. Appareil selon la revendication 1, comprenant en outre un capteur de flux (20) disposé dans le circuit de patient, dans lequel le dispositif de commande est structuré pour mesurer un volume des poumons en expansion du patient sur la base d'une sortie du capteur et du fait que la phase d'insufflation est terminée lorsque le volume des poumons en expansion atteint un certain niveau.

3. Appareil selon la revendication 1, dans lequel le certain état est une période de temps fixe prédéterminée s'écoulant après que le circuit de patient a été amené à entrer dans l'état opératoire.

4. Appareil selon la revendication 3, comprenant en outre un capteur de pression (30) structuré pour déterminer une pression sous-glottique du patient tandis que le patient est dans un état de plateau expiratoire, dans lequel le certain état est la pression sous-glottique qui dépasse un niveau de seuil prédéterminé.

5. Appareil selon la revendication 1, dans lequel le fait d'amener le circuit de patient à entrer dans l'état opératoire comprend le fait d'amener le composant générateur de flux de gaz à fournir un flux de gaz d'insufflation sous pression positive accru au patient, le flux de gaz d'insufflation sous pression positive accru ayant une pression qui est supérieure à une pression du flux de gaz d'insufflation sous pression positive fourni au cours de la phase d'insufflation.

6. Appareil selon la revendication 1, comprenant en outre une soupape (24) disposée dans le circuit de patient, dans lequel le fait d'amener le circuit de patient à entrer dans l'état opératoire comprend la fermeture de la soupape pour amener le circuit de patient à être au moins sensiblement physiquement occlus.

7. Appareil selon la revendication 1, dans lequel le composant générateur de flux de gaz comprend un générateur de flux de gaz sous pression positive (4) structuré pour générer le flux de gaz d'insufflation sous pression positive et un générateur de gaz sous pression négative séparé (6) structuré pour générer le flux de gaz d'exsufflation sous pression négative.

8. Appareil selon la revendication 1, dans lequel le composant générateur de flux de gaz comprend un générateur de flux de gaz (28) structuré pour générer à la fois le flux de gaz d'insufflation sous pression positive et le flux de gaz d'exsufflation sous pression négative.
